# EUROPEAN PATENT SPECIFICATION

(11) **EP 4 274 530 B1**
(45) Date of publication and mention of the grant of the patent: **10.06.2026**
(21) Application number: 22700582.4
(22) Date of filing: 06.01.2022
(51) Int. Cl.: A61F 13/00, A61F 13/02, A61M 1/00

(54) **NEGATIVE PRESSURE WOUND THERAPY SYSTEMS**
UNTERDRUCK-WUNDTHERAPIESYSTEME
SYSTÈMES DE THÉRAPIE DE PLAIES À PRESSION NÉGATIVE

(30) Priority: 06.01.2021 EP 21150385; 20.08.2021 EP 21192433
(43) Date of publication of application: 15.11.2023
(73) Proprietor: Sunmedic AB, 235 32 Vellinge (SE)
(72) Inventor: KARLADANI, Abbas, Hallaj, 216 31 Limhamn (SE); OZOOMCHI, Mehdi, Jafar, 236 32 Höllviken (SE); KARLADANI, Parisa, Hallaj, 211 42 Malmö (SE)
(74) Representative: Høiberg P/S
(86) International application number: PCT/EP2022/050185
(87) International publication number: WO 2022/148801

(56) References cited:
- WO-A1-2011/135287
- WO-A1-2017/114745
- WO-A1-2019/086330

## Description

The present disclosure relates to wound dressings, air connectors, and systems for use in negative pressure wound therapy, allowing for improved safety and efficacy with an improved ability to retain wound exudate within the dressing and a decreased risk of contamination.

### Background of invention

Negative-pressure wound therapy (NPWT), is a therapeutic technique using a suction pump, tubing and a dressing to remove excess exudate and promote healing in acute or chronic wounds, for example diabetic leg ulcers, lower leg wounds, surgical incision, traumatic wounds, burns, infected wounds, necrotizing fasciitis, infected sternal wounds and after skin grafting. The therapy involves the controlled application of subatmospheric pressure to the local wound environment for a duration that varies from a few days to months, depending on the treatment aim and the nature of the wound.

Typically a NPWT system comprises a dressing, a tube, a canister and a suction pump, wherein the canister is used to collect wound exudate in a way that ensures that the exudate does not reach the pump.

Alarm systems may be used in order to notify the user of the NPWT system that the dressing requires replacement, for example visual or audible indicators. The alarm systems may comprise sensors detecting environmental changes that occur upon exudate flow through the tubing. Canister systems typically have designs which allow for movement and rotation of the canister, while retaining evacuated exudate.

Canister-free systems have also been shown, wherein the wound exudate is to be retained by an absorbent within the dressing. For such systems it is important that the dressing is replaced regularly to ensure that the absorbent has not reached its maximum absorbent capacity, thereby avoiding exudate from reaching the pump unit.

The alarm systems for detecting contaminants within the systems typically give a good indication whether parts of the system have been contaminated, thereby requiring replacement. Indicators for change of dressing may assist in making a user aware of a need to replace the dressing, thereby decreasing the risk of contamination. Nonetheless, contamination control is always a concern when using a vacuum device on an open wound. Reducing the risk for potential contamination would allow for a more care-free use of the NPWT system, as it would require less maintenance, and in addition it would decrease the costs of the system.

WO 2011/135287 A1 discloses a wound dressing for applying a topical negative pressure at a wound site, comprising a gas impermeable cover layer comprising an orifice and a suction port sealed to the cover layer around a perimeter of the orifice, the port comprising a liquid impermeable, gas permeable filter element arranged to prevent a liquid from entering a connection portion for connection to a source of negative pressure.

WO 2019/086330 A1 discloses a wound dressing comprising a wound contacting layer, a transmission layer, a cover layer and a three dimensional filter element. The wound dressing may be connected to a source of negative pressure by a fluidic connector. WO 2017/114745 A1 discloses a wound dressing comprising an aperture in the absorbent layer underneath the fluidic connector.

### Summary of invention

The invention is defined by the claims. Any subject matter falling outside the scope of the claims is provided for information purposes only.

The present inventors have realized that negative pressure wound therapy systems may be configured in ways that increase their safety and efficacy. Specifically, the present inventors have realized how NPWT dressing and systems comprising improved configurations for contamination control may be achieved. Contamination control is one of the most important parameters of a NPWT system, allowing for improved safety and efficiency while enabling a more care-free device requiring less maintenance and dressing replacement.

The present disclosure therefore, in a first aspect, relates to a wound dressing for use in negative pressure wound therapy, the wound dressing comprising:
- a wound contacting layer comprising multiple perforations;
- a spacer layer, above the wound contacting layer, configured for transmission of wound exudate away from the wound site;
- an absorbent layer, above the spacer layer;
- a cover layer comprising a cover layer aperture, and wherein the cover layer is sealed to the wound contacting layer along a peripheral part of the wound dressing, thereby forming a sealed environment comprising the spacer layer and the absorbent layer;
- at least one sealing layer, sealed to the cover layer around the cover layer aperture, the at least one sealing layer comprising a sealing layer aperture, the sealing layer aperture being in fluidic communication with the cover layer aperture; and
- a filter element, located in, or below, the cover layer aperture and above the spacer layer, and wherein the filter element is retained by the sealing layer and arranged to cover the sealing layer aperture;
   wherein the absorbent layer comprises an absorbent layer aperture in fluidic connection with the cover layer aperture; and
   wherein the at least one sealing layer forms a conduit that extends from the part of the cover layer that is sealed to the sealing layer, towards the bottom of the absorbent layer through at least a part of the absorbent layer aperture.

The at least one sealing layer that is sealed to the cover layer around the cover layer aperture, and the at least one sealing layer comprises a sealing layer aperture, the sealing layer aperture being in fluidic communication with the cover layer aperture. Typically the sealing layer is sealed to the cover layer around the cover layer aperture and the sealing layer aperture.

The wound dressing comprises a filter element that is located in, or below, the cover layer aperture and above the spacer layer, and wherein the filter element is retained by the sealing layer and arranged to cover the sealing layer aperture.

The cover layer is sealed to the wound contacting layer along a peripheral part of the wound dressing, thereby forming a sealed environment comprising the spacer layer and the absorbent layer.

In an embodiment of the present disclosure, the filter element comprises, or consists of, a paper filter element with a pore size of 0.1 µm or less, such as 0.05 µm or less, or even 0.01 µm or less. The filter element is preferably configured such that wound exudate is prevented from crossing the filter element, thereby acting to retain the wound exudate within the sealed environment of the wound dressing. This ensures an increased contamination control and prevents wound exudate from reaching the negative pressure source.

The filter element is retained by the sealing layer, for example by an adhesive surface of the sealing layer, such that the filter element is located within, or below, the cover layer aperture.

Further, in another embodiment of the present disclosure, the absorbent layer comprises an absorbent layer aperture that extends vertically through said absorbent layer, i.e. from the cover layer to the wound contacting layer. In specific embodiments of the present disclosure, the filter element may be arranged below at least a part of the absorbent layer. For example, the filter element may be positioned level with the lower surface of the absorbent layer. In a preferred embodiment of the present disclosure, typically wherein the filter element is arranged such that it is below the upper surface of the absorbent layer, the sealing layer is arranged such that it extends from the cover layer towards the wound contacting layer, through at least a part of the absorbent layer.

The sealing layer aperture is typically located at a lower end of the sealing layer, facing the wound contacting layer. The filter element may be located to cover the sealing layer aperture, preferably wherein the filter element is located within the conduit. The sealing layer may thereby form a continuous liquid barrier extending from the cover layer to the bottom of the absorbent layer.

In an embodiment of the present disclosure, the sealing layer is formed in a material that is capable of blocking passage of wound exudate. The material of the sealing layer may for example comprise or consist of hydrocolloid, or a hydrophobic material.

In specific embodiments of the present disclosure, the sealing layer might be a layer of polyurethane or polycarbonate film or any other medical grade polymer film coated at least partially with one or a combination of medical grade synthetic rubber, or acrylate adhesive, or soft silicone gel, or polyurethane.

In specific embodiments of the present disclosure, the sealing layer might comprise a biological adhesive such as clotting proteins, for example fibrin. The sealing layer may in specific embodiments of the present disclosure be configured to restrict passage of fluids, preferably at least liquids, such as wound exudate.

In specific embodiments of the present disclosure, the sealing layer comprises an aperture that at least partially overlaps the cover layer aperture, and wherein the filter element is located between said apertures. Typically, wherein the filter element is larger than at least one of said apertures, preferably wherein the filter element is smaller than one of said apertures and larger than the other of said apertures. Preferably the centers of the sealing layer aperture, the cover layer aperture and the filter element are aligned, such as along a horizontal axis perpendicular to the wound contacting layer. For example, the sealing layer aperture, the cover layer aperture, and the filter element may be substantially aligned, and wherein the sealing layer aperture is smaller than the filter element while the cover layer aperture is larger than the filter element. The filter element may be arranged within the cover layer aperture and retained by the sealing layer.

The cover layer aperture and the sealing layer aperture is in fluid communication and arranged such that a sealed fluid flow path is formed between the two such that it allows for fluid communication between the sealed environment of the dressing, and the environment outside the dressing connected to the cover layer aperture, typically a negative pressure source.

In a further aspect, the present disclosure relates to a wound treatment apparatus for use in a canister-free negative pressure wound therapy, the wound treatment apparatus comprising:
- a wound dressing according to the invention;
- an air connector adapted to provide negative pressure, from a negative pressure source, to the wound dressing through the cover layer aperture, the air connector comprising:
   - a proximal end configured for connecting to a negative pressure source, and a distal end configured for connecting to the wound dressing;
   - a top layer and a bottom layer that are adhered together forming a sealed environment;
   - a connector spacer layer between the top and bottom layers;
   - an opening at the distal end that is positioned above the cover layer aperture, such that a sealed flow channel between the sealed environment of the dressing and the sealed environment of the connector is formed.

In a specific embodiment of the present disclosure, a surface surrounding the opening at the distal end is adhered to a surface surrounding the cover layer aperture. Preferably in such a way that a sealed fluid connection between the sealed environment of the dressing and the sealed environment of the air connector is formed. In a further embodiment of the present disclosure, a sealed fluid connection is formed between the sealed environment of the dressing and the sealed environment of the air connector, for example wherein said fluid connection comprises the sealing layer aperture, the cover layer aperture and the filter element. Preferably wherein said fluid connection further comprises the fluidic conduit formed by the sealing layer.

In a further embodiment of the present disclosure, the apparatus further comprises a negative pressure source, arranged to provide negative pressure to the dressing through the air connector. Preferably, the wound treatment apparatus is, or forms part of, a single-use canister-free NPWT system.

### Description of drawings

**Fig. 1A-B** show a wound dressing comprising a sealing layer and a filter element according to a specific embodiment of the present disclosure
**Fig. 2A-B** show a wound dressing comprising a sealing layer and a filter element according to a specific embodiment of the present disclosure
**Fig. 3A-B** show a wound dressing comprising multiple sealing layers and a filter element comprising multiple layers according to a specific embodiment of the present disclosure
**Fig. 4A-B** show a wound dressing comprising a sealing layer, and a filter element, and an absorbent layer aperture according to a specific embodiment of the present disclosure
**Fig. 5A-B** show a wound dressing comprising a sealing layer, and a filter element, and an absorbent layer aperture according to a specific embodiment of the present disclosure
**Fig. 6** shows an air connector according to a specific embodiment of the present disclosure
**Fig. 7** shows a wound treatment apparatus according to a specific embodiment of the present disclosure
**Fig. 8** shows a top layer and a bottom layer of the spacer layer according to a specific embodiment of the present disclosure

### Detailed description of the invention

In a first aspect, the present disclosure relates to a wound dressing for use in negative pressure wound therapy, the wound dressing comprising:
- a wound contacting layer comprising multiple perforations;
- a spacer layer, above the wound contacting layer, configured for transmission of wound exudate away from the wound site;
- an absorbent layer, above the spacer layer;
- a cover layer comprising a cover layer aperture, and wherein the cover layer is sealed to the wound contacting layer along a peripheral part of the wound dressing, thereby forming a sealed environment comprising the spacer layer and the absorbent layer;
- at least one sealing layer, sealed to the cover layer around the cover layer aperture, the at least one sealing layer comprising a sealing layer aperture, the sealing layer aperture being in fluidic communication with the cover layer aperture; and
- a filter element, located in, or below, the cover layer aperture and above the spacer layer, and wherein the filter element is retained by the sealing layer and arranged to cover the sealing layer aperture;
   wherein the absorbent layer comprises an absorbent layer aperture in fluidic connection with the cover layer aperture; and
   wherein the at least one sealing layer forms a conduit that extends from the part of the cover layer that is sealed to the sealing layer, towards the bottom of the absorbent layer through at least a part of the absorbent layer aperture.

In an embodiment of the present disclosure, the wound contacting layer is configured for contacting a wound surface and/or tissue surrounding a wound surface. Preferably, the wound contacting layer is a layer that has a low adherence to wound tissue and/or tissue surrounding a wound. The wound contacting layer may thereby be a non-adhering layer. Preferably, the wound contacting layer is a flexible and conformable layer, for example a perforated layer of silicon, polyurethane layer or polyethylene. The wound contacting layers typically comprises a number of apertures forming a fluidic communication between the inside of the dressing, e.g. the dressing sealed environment, and the wound. Fluids, such as wound exudate, may flow through the apertures of the wound contacting layer into the sealed environment of the dressing.

The spacer layer is preferably porous, and located above the wound contacting layer. This porous layer, or spacer layer, allows for transmission of fluids, including liquids and gases, away from a wound site into the upper layers of the wound dressing. In particular, the spacer layer ensures that an open air channel can be maintained to communicate negative pressure over the wound area even when the absorbent layer has absorbed substantial amounts of exudates. The spacer layer should remain open under the typical pressures that will be applied during negative pressure wound therapy as described above, so that the whole wound site sees an equalized negative pressure. The layer may be formed of a material having a three-dimensional structure, such as a knitted or woven spacer fabric. Preferably said spacer fabric comprises a top layer and a bottom layer and a number of filaments extending substantially vertically, connecting the top layer and the bottom layer. It is a preference that the top layer and the bottom layer comprises a number of apertures and that the sizes of the holes, on both layers, are substantially the same. In some embodiments, the size of the holes on the top and bottom layers of the spacer layer is larger than 500 µm. In some embodiments the size of the holes, on both the top layer and bottom layer, of the spacer layer, may be between 500 µm and 800 µm, or between 800 µm and 1400 µm, or preferably between 1400 µm and 2000 µm.

Similar sized holes of the top layer and the bottom layer of the spacer layer may have multiple advantages. For example, it is given that a larger size of the holes facing the wound contacting layer offer a greater chance to allow semi-solid particles released from sluff, biofilm and other harmful material to be separated from the wound surface and pass through the spacer layer into an upper layer of the wound dressing, such as the absorbent layer. This is in contrast to a spacer layer in which its bottom layer has smaller holes compared to the holes at its top layer.

Additional advantages of a spacer layer with similar sized holes is that particulate matter, such as blood agglutinates, does not become retained within the spacer layer. In a preferred embodiment of the present disclosure, the spacer layer is of polyester, such as a 3D mesh polyester fabric.

In an embodiment of the present disclosure, the wound dressing comprises an absorbent layer configured for retaining wound exudate. The absorbent layer may comprise a number of layers comprising super absorbent particles. In order to reduce the thickness of the absorbent layer, the absorbent layer may comprise one or more layers comprising welded layers of absorbent materials, such as a super absorbent material. In specific embodiments of the present disclosure, the absorbent layer comprises two super absorbent layers. In certain embodiments the absorbent layer comprises three or four layers comprising absorbent material. It is a preference that the absorbent layer is directly below the cover layer. It is a preference that the absorbent layer comprises granulated super absorbent particles.

Super-absorber particles/fibers may be, for example, sodium polyacrylate or carbomethoxycellulose materials or the like or any material capable of absorbing many times its own weight in liquid. In some embodiments, said material can absorb more than five times its own weight of 0.9 percent W/W saline, etc. In some embodiments, the super absorbent material can absorb more than 15 times its own weight of 0.9 percent W/W saline, etc. In some embodiments, the super absorbent material is capable of absorbing more than 20 times its own weight of 0.9 percent W/W saline, etc. In an embodiment of the present disclosure, the super absorbent material is capable of absorbing more than 30 times its own weight of 0.9 percent W/W saline. In a preferred embodiment of the present disclosure, the super absorbent material is capable of absorbing more than 60 times its own weight of 0.9 percent W/W saline.

In a preferred embodiment the dressing comprises a cover layer in a liquid impermeable and gas permeable material. For example, the material of the cover layer may be polyurethane. The cover layer may comprise an adhesive for contacting the layer below the cover layer, such as the absorbent layer. In a preferred embodiment of the present disclosure, the cover layer comprises an aperture, i.e. a cover layer aperture. In specific embodiments of the present disclosure, the cover layer aperture is aligned with an absorbent layer aperture, such as aligned around a vertical axis, such as an axis perpendicular to the plane of the dressing. Thereby, the cover layer aperture may be centered around a vertical axis perpendicular to the plane of the dressing. In general embodiments of the present disclosure, the cover layer aperture is aligned with the filter element, and the sealing layer aperture, such as wherein each of the filter element, the cover layer aperture and the sealing layer aperture are centered around an axis perpendicular to the plane of the dressing.

In a further embodiment of the present disclosure, the dressing comprises a sealing layer adhered to the cover layer, such as the top surface of the cover layer facing away from the wound contacting layer, or a bottom surface of the cover layer facing the wound contacting layer. The sealing layer comprises a sealing layer aperture, the sealing layer aperture being in fluidic communication with the cover layer aperture. The sealing layer may further comprise adhesives, or the sealing layer may be provided in an adhering material, to adhere to other layers of the dressing.

The dressing comprises a filter element, arranged to cover the sealing layer aperture. The filter element is preferably impermeable to liquids, but permeable to gases. Preferably the filter element is provided to act as a liquid barrier and to ensure that no liquids are able to escape from the wound dressing. The filter element may also function as a bacterial barrier. The filter element may be provided with a pore size of 0.2 µm or less, such as 0.1 µm or less, such as 0.05 µm or less, or even 0.01 µm or less. In a preferred embodiment of the present disclosure, the filter element is provided in a paper material. The filter element can be attached or sealed to the cover layer and/or sealing layer. Preferably, the filter is arranged to cover the sealing layer aperture. The filter may be provided in a circular, or oval shape, for example a circular shape with a diameter of between 5 mm and 40 mm, such as between 7 mm and 30 mm, such as between 9 mm and 25 mm, such as between 10 mm and 20 mm, such as around 13 mm.

### Filter element

In an embodiment of the present disclosure, the filter is provided in a shape that is similar or identical to the shape of the sealing layer aperture and/or the cover layer aperture. Preferably the filter is provided in the same shape as the sealing layer aperture and the cover layer aperture. In a further embodiment of the present disclosure, the sealing layer aperture and the cover layer aperture are arranged such that they form a sealed connection, and the filter element is arranged such that it filters fluid that passes through said sealed connection. In a preferred embodiment of the present disclosure the filter element is provided in a size, i.e. a planar surface area, that is smaller than the size of the cover layer aperture, and larger than the size of the sealing layer aperture. In a specific embodiment of the present disclosure, the filter element may be provided in a size that is larger than both the cover layer aperture and the sealing layer aperture. Preferably, the filter element is arranged to cover or span the cover layer aperture and/or the sealing layer aperture, preferably, the filter element is arranged to seal the cover layer aperture and/or the sealing layer aperture.

In an embodiment of the present disclosure, the filter element is a paper filter element, preferably with a pore size of 0.1 µm or less, such as 0.05 µm or less, or 0.01 µm or less. In an embodiment of the present disclosure, the average pore size is between 0.01 µm and 1 µm, more preferably between 0.05 µm and 0.5 µm, yet more preferably between 0.1 µm and 0.4 µm such as around 0.3 µm.

The filter element may be arranged in different ways, but still allow for filtering of fluids before said fluids are allowed to escape from the wound dressing through the cover layer aperture. Preferably the filter element is configured to retain liquids within the wound dressing while gasses are allowed to pass through the filter element. In an embodiment of the present disclosure, the filter element is adhered to the sealing layer, such as being held in place by the sealing layer. The sealing layer may be provided in an adhesive material or the sealing layer may comprise an adhesive for adhering the filter element to the sealing layer. The filter element may be provided on a top surface of the sealing layer, such as a surface of the sealing layer faced away from the wound contacting layer. In an alternative embodiment, the filter element may be provided on a bottom surface of the sealing layer, such as a surface of the sealing layer faced towards the wound contacting layer. Typically, if the filter element is retained or adhered to a surface of the sealing layer that is above the cover layer, said surface is a bottom surface of the sealing layer. However, if the filter element is retained or adhered to a surface of the sealing layer that is below the cover layer, said surface is a top surface of the sealing layer. For example, if the sealing layer is provided as a planar element provided directly on top of the cover layer, the filter element is retained by, such as adhered to, a bottom surface of the sealing layer, typically such that at least a part of the filter element is within the cover layer aperture. The filter element may in such instance have a top surface that is substantially parallel with a top surface of the cover layer.

However, in alternative embodiment of the present disclosure, for example wherein the sealing layer is arranged such that it forms a conduit through the absorbent layer, it may be a preference to provide the filter element on a top surface of the sealing layer. The sealing layer may thereby be provided within the conduit, and the sealing layer aperture may be formed below the filter element, preferably with a size smaller than the filter element.

The absorbent layer comprises an absorbent layer aperture located below the cover layer aperture. Preferably the cover layer aperture covers at least a part of the absorbent layer aperture. In a preferred embodiment of the present disclosure, the cover layer aperture covers the absorbent layer aperture. Preferably, the size, i.e. the width, of the absorbent layer aperture is larger than the cover layer aperture. The sealing layer may be attached, such as adhered, to a top surface of the cover layer, or a bottom surface of the cover layer, and extend, in the shape of a conduit, downwards through the absorbent layer conduit. Preferably, a filter is located at a bottom end of the absorbent layer aperture, such as wherein the filter is attached to a top surface, or a bottom surface, of the sealing layer. The sealing layer thereby may form a sealed connection between the sealing layer aperture, and the cover layer aperture, preferably through the filter element.

In an embodiment of the present disclosure, the filter element is located at the same level, or below the cover layer. The filter element may in specific embodiments of the present disclosure, be arranged such that it is located within the cover layer aperture, such wherein the top surface of the filter element is aligned with a top surface of the cover layer. In other embodiments of the present disclosure, the filter element may be provided below the cover layer, such as wherein the filter element is located towards a bottom end of a sealing layer conduit. When the filter element is located in the cover layer aperture, such as wherein an upper surface of the filter element is substantially parallel with an upper surface of the cover layer aperture, the filter element may have substantially the same size and shape as the cover layer aperture. By positioning the filter element in the cover layer aperture, leakages may be prevented, in part because the total area that may be subject to leakages is smaller with respect to a filter element arranged to cover the cover layer aperture and sealing a filter element in the .

By positioning the filter element below the cover layer aperture, preferably directly below the cover layer aperture, the negative pressure will act to retain the filter element in its position, and the risk of device failure by movements of the filter element (e.g. by the applied negative pressure) is reduced. Thereby, by having a filter element that is sized and shaped similar to the cover layer aperture, or by having the filter element or the layer retaining the filter element (e.g. the sealing layer) sized and shaped to cover the cover layer aperture, the filter element may be held in position, and form an efficient seal to minimize leakages of wound exudate.

In specific embodiments of the present disclosure, the filter element is provided below at least a part of the absorbent layer. The filter layer may for example be provided below the absorbent layer, such as between the absorbent layer and the spacer layer. Alternatively, the filter element may be provided such that the bottom surface of the filter element is aligned with the bottom surface of the absorbent layer. In specific embodiments of the present disclosure, the filter element is provided within the absorbent layer. For example, the absorbent layer may comprise an absorbent layer conduit that only extends partially through the absorbent layer, such as from the top surface of the absorbent layer.

In an embodiment of the present disclosure, the filter element comprises a bottom surface facing the wound contacting layer and a top surface facing away from the wound contacting layer. It is a preference that the properties of the two planar surfaces of the filter have different structural properties. In a preferred embodiment of the present disclosure, the filter element has a first side and a second side, wherein the first side has a smoother surface than said second side. It is a preference that the filter element is arranged such that the smoother side, i.e. the first side, faces away from the wound contacting layer. In this way, water vapour that is transported away from the dressing will travel through the filter element from the smooth side to the rough, second side. This has shown to more efficiently block liquids from being transported through the filter, while maintaining an efficient transportation rate of the water vapour. Preferably, the filter element is arranged such that the sealing layer is arranged to retain the filter element by contacting the first side of the filter element. Thereby, it is a preference that the sealing layer is arranged to retain the filter element by at least contacting the first side of the filter element, e.g. by adhering to the first side of the filter element.

The difference in roughness of the filter element may be for example due to different pore sizes of the two sides. The roughness may for example be quantified by the arithmetical mean deviation of the assessed profile (Ra value). The smooth side (first side) may for example have a pore size that is below 0.1 µm and typically below 0.05 µm, commonly between 0.01 µm and 0.1 µm. The rough side (second side) may for example have a pore size that is above 0.1 µm and typically above 0.5 µm, commonly between 0.2 µm and 100 µm. Therefore, it is a preference that the filter element has a first side with a pore size between 0.01 and 0.1 µm and a second side with a pore size between 0.2 and 100 µm. Upon inspection of the filter element, it can typically be seen that the smooth side is shiny, while the rough side is dull.

In other embodiments of the present disclosure, the filter element comprises multiple filters, for example filters having differing hydrophilic-hydrophobic properties. Therefore, the filter element may comprise a first filter that is more hydrophilic and a second filter that is more hydrophobic, allowing for decreased clogging of the filter element. The filters of the filter element may be attached to each other, or spaced apart, such as spaced apart within the conduit formed by the sealing layer according to a specific embodiment of the present disclosure.

In specific embodiments of the present disclosure, the wound dressing comprises multiple sealing layers, such as two sealing layers. In the case of two sealing layers, the wound dressing may comprise a top sealing layer and a bottom sealing layer located at different levels, i.e. heights, of the wound dressing. For example, the top sealing layer may be attached to the top surface of the cover layer, and the bottom sealing layer may be attached to the bottom surface of the cover layer. It is a preference that each sealing layer comprises a sealing layer aperture and that each sealing layer aperture is in fluid communication. In a preferred embodiment the top and bottom sealing layers may be arranged to retain a filter element comprising one or more filter elements. For example, the filter element may be encapsulated by the top and bottom sealing layers, and wherein the sealing layer apertures of said layers are in fluid communication, with the filter element in between said apertures. The filter element may be arranged to filter fluids, typically to block liquids, from passing through the filter. The filter element may thereby prevent liquids from reaching the top sealing layer aperture. In a preferred embodiment, the filter element comprises a filter in a hydrophilic material, such as a super absorbent polymer, laminated between two filters in a hydrophobic material. It is a preference that the three filters have the same size and shape, however, the hydrophobic filters may in certain embodiments extend beyond the edge of the hydrophilic filter. The filter element may in this way be configured to restrict the fluid path, such that a connected sensor can decrease the restriction, and thereafter warn a user that the wound dressing requires replacement. Any sensor may be used that is capable of detecting the pressure within the system. The system may therefore be configured to detect pressure changes or the lack of pressure changes during pumping, and as a result warn the user of a blockage in the tubing, and that the wound dressing needs to be replaced. Typically the wound dressing may comprise a filter element with at least a hydrophilic part, such as comprising super absorbent material, however in alternative embodiments, the same function may be achieved by the use of a filler unit in a hydrophilic material, such as superabsorbent polymers. The filler unit is typically located in the conduit formed by the sealing layer extending downwards from the cover layer. The filler unit may be a hydrophilic material such as polyvinyl alcohol or super absorbent polymer. However, in other embodiments of the present disclosure the filler unit may comprise or consist of a hydrophobic material. In this way the wound dressing may be configured for prolonged operation due to a possibility of prolonged supply of negative pressure.

In certain embodiments of the present disclosure the filter element may comprise a function releasing block alarm, in which it is used two hydrophobic filter layers separated by a hydrophilic layer. The first filter layer is attached to the bottom surface of the sealing layer positioned in the cover layer aperture facing wound contacting layer. The second filter layer is attached to the top surface of a second sealing layer and attached to the bottom layer of the cover layer facing away from the wound contacting layer. These two filters may be positioned within, and fill, the cover layer aperture, and are separated by a hydrophilic material, i.e. a hydrophilic layer, such as a super absorbent polymer (SAP).

The filter element may be attached, such as adhered, to either side of the cover layer or the sealing layer. In an embodiment of the present disclosure, the filter element is attached, such as adhered, to a top surface or a bottom surface of the sealing layer. As disclosed elsewhere herein, it may be a preference that the filter element is attached to a part of the top surface of the sealing layer when the filter element is attached, such as adhered, to a part of the surface of the sealing layer that is below the cover layer. It may further be a preference that the filter element is attached to a part of the bottom surface of the sealing layer when the filter element is attached, such as adhered, to a part of the surface of the sealing layer that is above the cover layer.

### Sealing layer

The sealing layer is preferably provided in a material that is impermeable to air and water vapour (i.e. non-breathable), however the sealing layer may be impermeable to gasses in general. In addition, the sealing layer may be liquid impermeable, preferably impermeable to at least water. Thereby, the sealing layer may be gas and liquid impermeable. However, it is a preference that the sealing layer is impermeable to at least air and water vapour, preferably also water. The sealing layer is preferably non-breathable.

The sealing layer is preferably arranged as an occlusive wall for preventing the passage of at least air and water vapour, contrary to for example a conventional filter of a wound dressing. In this way, the absorbent properties of the wound dressing will be controlled by the sealing layer, as the wound dressing will be arranged such that at least air and water vapour, preferably as well as other gasses and/or liquids will be diverted towards the sealing layer aperture. Further, a water and air impermeable sealing layer will act to decrease the risk of leakages.

The sealing layer may for example comprise or consists of a hydrophobic material. Preferably, the sealing layer comprises or consists of hydrocolloid or hydrophobic polyurethane. In an embodiment of the present disclosure, the sealing layer comprises or consists of a hydrophobic material. In specific embodiments of the present disclosure, the material of the sealing layer is hydrocolloid or hydrophobic polyurethane. It is known that these materials, at relatively small thicknesses, may be breathable. As it is a preference that the sealing layer is non-breathable, it may be a requirement to provide the sealing layer at a sufficient thickness such that the sealing layer is non-breathable and/or impermeable to air and water vapour.

Preferably the sealing layer is flexible, waterproof and self-adhering, for example the sealing layer may be a hydrocolloid. As the sealing layer is self-adherent it decreases the risk for leakages. The sealing layer is preferably biodegradable and non-breathable. The sealing layer may comprise or consists of a hydrocolloid arranged such that the layer absorbs wound exudate and forms a gel that is impermeable to air and water vapour, preferably as well as other liquids, such as at least water.

Hydrocolloids may have various compositions, such as around 40% polyisobutylene, 20% sodium carboxymethylcellulose, 20% gelatin and 20% pectin. Typically, the hydrocolloid absorbs wound fluid and as a result changes into a jelly-like mass. The outside of the dressing is covered with a polyurethane foam or film which enables the exchange of water vapor and protects the wound against contamination from the outside. Hydrocolloids are moisture-retentive materials, which typically contain gel-forming agents such as sodium carboxymethylcellulose and gelatin. Many hydrocolloids combine the gel-forming properties with elastomers and adhesives thereby forming an absorbent, self-adhesive, and liquid-impermeable film. In a specific embodiment of the present disclosure, the sealing layer comprises hydrocolloids, elastomers, and/or adhesives. In an embodiment of the present disclosure, the sealing layer is a self-adhesive and liquid impermeable film. In specific embodiments of the present disclosure, at least one side of the sealing layer may comprise a polyurethane foam or film. For example, wherein a part of the sealing layer forms a conduit between the sealing layer aperture and the cover layer aperture, the one side of said conduit, typically the inside, may be provided with a polyurethane foam or film. In specific embodiments of the present disclosure, the sealing layer may be provided in a fluid-impermeable material, configured for blocking passage of liquids and gasses. This may allow for supplying negative pressure to the wound dressing through the sealing layer aperture only. In other embodiments of the present disclosure, the sealing layer may be provided in a liquid-impermeable and gas permeable material (such as a breathable material). If the sealing layer is provided in a material that is permeable to gasses, the evaporation of liquids within the wound dressing may be accelerated, thereby allowing for a larger capacity of the wound dressing and it can consequently operate for a longer time before being required to be replaced.

In a preferred embodiment of the present disclosure, the at least one sealing layer is sealed to the cover layer around the cover layer aperture, and around the sealing layer aperture of the at least one sealing layer, such that the sealing layer aperture is in fluid communication with the cover layer aperture. The seal is thereby formed around the sealing layer aperture and the cover layer aperture. In specific embodiments of the present disclosure, the filter element is located between the sealing layer aperture and the cover layer aperture.

In specific embodiments of the present disclosure, the wound dressing comprises multiple sealing layers, such as two sealing layers. In the case of two sealing layers, the wound dressing may comprise a top sealing layer and a bottom sealing layer located at different levels, i.e. heights, of the wound dressing. For example, the top sealing layer may be attached to the top surface of the cover layer, and the bottom sealing layer may be attached to the bottom surface of the cover layer. It is a preference that each sealing layer comprises a sealing layer aperture and that each sealing layer aperture is in fluid communication. In a preferred embodiment the top and bottom sealing layers may be arranged to retain a filter element comprising one or more filter elements. For example, the filter element may be encapsulated by the top and bottom sealing layers, and wherein the sealing layer apertures of said layers are in fluid communication, with the filter element in between said apertures. The filter element may be arranged to filter fluids, typically to block liquids, from passing through the filter. The filter element may thereby prevent liquids from reaching the top sealing layer aperture.

In specific embodiments of the present disclosure, an adhesive layer has been applied to the sealing layer, on one or both sides of the sealing layer, for attaching to the cover layer and/or the filter element. Thereby, at least one side of the sealing layer may comprise an adhesive, preferably both sides.

It is a preference that the wound dressing comprises two sealing layers, a top sealing layer and a bottom sealing layers, and wherein the top sealing layer is arranged to adhere to an outer part of the cover layer, i.e. a part outside of the sealed environment of the wound dressing, and a bottom sealing layer that is arranged to adhere to an inner part of the cover layer, i.e. at a part inside the sealed environment of the wound dressing. This arrangement has shown to lead to a significant reduction in the risk of leakages, and the durability of the wound dressing. Depending on where on the patient the dressing is located, the dressing may be subjected to movements. For example, if the wound is on the knee, elbow or another joint, the dressing will be moved constantly, and a strong and durable seal is required in order to maintain full functionality of the wound dressing.

The sealing layer comprises an aperture. The sealing layer aperture preferably forms a through-hole through the sealing layer. It is a preference that the sealing layer aperture is covered by the cover layer aperture, such as wherein the cover layer aperture and the sealing layer aperture is centered around the same vertical axis. In an embodiment of the present disclosure, the sealing layer aperture has the same shape as the cover layer aperture. Typically, the sealing layer aperture, and/or the cover layer aperture are oval or circular, however in specific embodiments of the present disclosure, said apertures may be provided in any shape such as irregular or regular, for example a polygonal shape.

In an embodiment of the present disclosure, the sealing layer aperture is smaller than the cover layer aperture. It may be a preference that the sealing layer is arranged to retain the filter element, and that a filter of the filter element directly covers the sealing layer aperture. Typically, the sealing layer is therefore smaller, i.e. has a smaller diameter or width, than the diameter or width of the filter element. The cover layer aperture may be provided with an identical size, such as diameter, as the sealing layer, however, the cover layer aperture may be larger, i.e. the size, such as the diameter may be larger. Therefore, in an embodiment of the present disclosure, the cover layer aperture substantially covers the sealing layer aperture.

The sealing layer may be provided in a self-adhesive material, or may comprise one or more adhesive layers on at least a part of one or both sides of the sealing layer. In an embodiment of the present disclosure, the sealing layer adheres to a part of the cover layer surrounding the cover layer aperture. For example, the sealing layer may be attached, such as adhered, to a part of the top surface of the cover layer, i.e. the surface facing away from the wound contacting layer. In other embodiments of the present disclosure, the sealing layers are attached, such as adhered, to a part of the bottom surface of the cover layer. The sealing layer is attached around the cover layer aperture, such that the sealing layer is sealed to the cover layer around the cover layer aperture. It is a preference that the sealing layer aperture is located inside the part of the sealing layer that is sealed to the cover layer. The sealing layer may for example be sealed to the cover layer aperture in a ring pattern around the cover layer aperture, while the sealing layer aperture is located inside said ring pattern on the sealing layer.

The cover layer and the wound contacting layer forms a sealed environment comprising the spacer layer and the absorbent layer. The wound dressing comprises a number of fluid channels for fluid communication between the sealed environment of the wound dressing and an environment outside the wound dressing, such as a negative pressure source of a wound site. The fluid channels comprises or consists of a number of perforations in the wound contacting layer, and one or more fluid channels through the cover layer. In a preferred embodiment of the present disclosure, the wound dressing has a single fluid channel through the cover layer, said fluid channel comprises one or more, such as two, sealing layer apertures, the filter element, the cover layer aperture, and optionally a filler unit. It is a preference that the filter element is arranged such that it extends across the entire fluid channel, such that fluid is required to pass the filter element, and optionally a filler unit, in order to reached an environment outside the wound dressing, through the one or more sealing layer apertures, and the cover layer apertures.

In an embodiment of the present disclosure, the sealing layer extends from the cover layer towards the wound contacting layer, through the absorbent layer. The sealing layer may be sealed around the cover layer aperture, for example in a ring pattern, and have a sealing layer aperture within the area sealed to the cover layer. The sealing layer may extend towards the wound contacting layer. In an embodiment of the present disclosure, the sealing layer extends from the cover layer towards the wound contacting layer, through one or more layers of the wound dressing. For example, the sealing layer may extend from the cover layer through apertures in one or more layers of the wound dressing. In a particular embodiment of the present disclosure, the sealing layer extends from the cover layer towards the wound contacting layer through an absorbent layer aperture, and wherein the sealing layer is arranged such that the sealing layer forms a conduit within the absorbent layer aperture. In an embodiment of the present disclosure, the sealing layer is arranged to form a conduit that is impermeable to at least air and water vapour, preferably also liquids. The conduit preferably extends through at least a part of the absorbent layer aperture, such as from the cover layer aperture to the spacer layer, or from the cover layer aperture to a part of the absorbent layer aperture. Similar to other apertures mentioned herein, also the absorbent layer aperture may be a through hole, through the absorbent layer. However, the absorbent layer aperture does not have to go entirely through the absorbent layer, in specific embodiments the absorbent layer aperture penetrates a part of the absorbent layer, typically from the top side, facing away from the wound contacting layer, and through a part of the absorbent layer. In a preferred embodiment of the present disclosure, the sealing layer forms a continuous liquid barrier extending from the cover layer to the bottom of the absorbent layer. In certain embodiments of the present disclosure, the absorbent layer is intact (meaning that that the absorbent layer does not include an absorbent layer aperture) and the sealing layer top surface is attached to the bottom surface of the cover layer and to the top surface of the absorbent layer.

The sealing layer aperture is in fluid communication with the cover layer aperture, in addition the sealing layer is sealed around the cover layer aperture wherein the sealing layer aperture is located inside the sealed area of the sealing layer, for example inside a sealed area in the form of a ring pattern.

In an embodiment of the present disclosure, the sealing layer aperture and/or the cover layer aperture is configured to provide fluid communication between the inside of the dressing and the outside of the dressing, such as a negative pressure source. Typically an air connector is attached to the cover layer such that the connector is capable of fluid communication with the inside of the dressing, through the cover layer aperture and the sealing layer aperture. Thereby, in an embodiment of the present disclosure, the wound dressing is configured such that the sealing layer aperture and the cover layer aperture form a sealed connection between the sealed environment of the wound dressing, i.e. the sealed environment formed by the sealing of the cover layer and the wound contacting layer, and an opening that is suitable for receiving an air connector, an opening for communicating with a negative pressure source, or an opening of the top side of the wound dressing, i.e. an opening in the cover layer.

In a specific embodiment of the present disclosure, the sealing layer is liquid impermeable and gas permeable, thereby allowing for removal water vapor while ensuring that liquid is retained by the wound dressing,

### Additional items

The cover layer is sealed to the wound contacting layer, such as at a peripheral part of the dressing, such that a sealed environment containing the spacer layer and the absorbent layer is formed. The cover layer may for example be welded to the wound contacting layer along a perimeter, as seen from above, of the wound dressing. Thereby, the wound dressing may encapsulate the absorbent layer and the spacer layer and typically have a single fluid aperture for fluid communication with the outside environment, typically a pressure source, although specific embodiments of the present disclosure comprises multiple apertures in the cover layer. In addition, the bottom layer of the dressing, i.e. the wound contacting layer typically comprises multiple apertures for fluid communication with the wound.

In an embodiment of the present disclosure, the spacer layer is configured to wick fluid away from the wound contacting layer. The spacer layer is preferably a 3D mesh polyester fabric, comprising a top fabric layer and a bottom fabric layer connected by a number of filaments. It is a preference that the holes of the top layer and the bottom layer are substantially the same size, such as the same size. The spacer layer is preferably porous, and located above the wound contacting layer. This porous layer, or spacer layer, allows for transmission of fluid including liquid and gas away from a wound site into upper layers of the wound dressing. In particular, the spacer layer ensures that an open air channel can be maintained to communicate negative pressure over the wound area even when the absorbent layer has absorbed substantial amounts of exudates. The spacer layer should remain open under the typical pressures that will be applied during negative pressure wound therapy as described above, so that the whole wound site sees an equalized negative pressure. The layer may be formed of a material having a three-dimensional structure, such as a knitted or woven spacer fabric. Preferably said spacer fabric comprises a top layer and a bottom layer and a number of filaments extending substantially vertically, connecting the top layer and the bottom layer. It is a preference that the top layer and the bottom layer comprises a number of apertures and that the sizes of the holes are substantially the same. In some embodiments of the present disclosure, the size of the holes on the top and bottom layers of the spacer layer is larger than 500 µm. In some embodiments the size of the holes on the top layer and the bottom layer of the spacer layer may be between 500 µm and 800 µm, or between 800 µm and 1400 µm, or preferably between 1400 µm and 2000 µm.

Similar sized holes of the top layer and the bottom layer of the spacer layer may have multiple advantages. For example, it is given that a larger size of the holes facing the wound contacting layer offer a greater chance to allow semi-solid particles released from sluff, biofilm and other harmful material to be separated from the wound surface and pass through the spacer layer into an upper layer of the wound dressing, such as the absorbent layer. This is in contrast to a spacer layer in which its bottom layer has smaller holes compared to the holes at its top layer.

Additional advantages of a spacer layer with similar sized holes is that particulate matter, such as blood agglutinates, does not become retained within the spacer layer. In a preferred embodiment of the present disclosure, the spacer layer is of polyester, such as a 3D mesh polyester fabric.

### Wound treatment apparatus

In a further aspect, the present disclosure relates to a a wound treatment apparatus (15) for use in a canister-free negative pressure wound therapy, the wound treatment apparatus (15) comprising:
- a wound dressing (1) according to the invention;
- an air connector (16) adapted to provide negative pressure, from a negative pressure source, to the wound dressing (1), through the cover layer aperture (4), the air connector (16) comprising:
   - a proximal end (17) configured for connecting to a negative pressure source, and a distal end (18) configured for connecting to the wound dressing (1);
   - a top layer (19) and a bottom layer (20) that are adhered together forming a sealed environment;
   - a connector spacer layer (21) between the top and bottom layers (19, 20);
   - an opening (22) at the distal end (18) that is positioned above the cover layer aperture (4), such that a sealed flow channel between the sealed environment of the dressing and the sealed environment of the air connector is formed.

In an embodiment of the present disclosure, the wound contacting layer is configured for contacting a wound surface and/or tissue surrounding a wound surface. Preferably, the wound contacting layer is a layer that has a low adherence to wound tissue and/or tissue surrounding a wound. The wound contacting layer may thereby be a non-adhering layer. Preferably, the wound contacting layer is a flexible and conformable layer, for example a perforated layer of silicon, polyurethane layer or polyethylene. The wound contacting layers typically comprises a number of apertures forming a fluidic communication between the inside of the dressing, e.g. the dressing sealed environment, and the wound. Fluids, such as wound exudate, may flow through the apertures of the wound contacting layer into the sealed environment of the dressing.

The spacer layer is preferably porous, and located above the wound contacting layer. This porous layer, or spacer layer, allows for transmission of fluids, including liquids and gases, away from a wound site into the upper layers of the wound dressing. In particular, the spacer layer ensures that an open air channel can be maintained to communicate negative pressure over the wound area even when the absorbent layer has absorbed substantial amounts of exudates. The spacer layer should remain open under the typical pressures that will be applied during negative pressure wound therapy as described above, so that the whole wound site sees an equalized negative pressure. The layer may be formed of a material having a three-dimensional structure, such as a knitted or woven spacer fabric. Preferably said spacer fabric comprises a top layer and a bottom layer and a number of filaments extending substantially vertically, connecting the top layer and the bottom layer. It is a preference that the top layer and the bottom layer comprises a number of apertures and that the sizes of the holes, on both layers, are substantially the same. In some embodiments, the size of the holes on the top and bottom layers of the spacer layer is larger than 500 µm. In some embodiments the size of the holes on spacer top layer and the bottom layer of the spacer layer may be between 500 µm and 800 µm, or between 800 µm and 1400 µm, or preferably between 1400 µm and 2000 µm.

Similar sized holes of the top layer and the bottom layer of the spacer layer may have multiple advantages. For example, it is given that a larger size of the holes facing the wound contacting layer offer a greater chance to allow semi-solid particles released from sluff, biofilm and other harmful material to be separated from the wound surface and pass through the spacer layer into an upper layer of the wound dressing, such as the absorbent layer. This is in contrast to a spacer layer in which its bottom layer has smaller holes compared to the holes at its top layer.

Additional advantages of a spacer layer with similar sized holes is that particulate matter, such as blood agglutinates, does not become retained within the spacer layer. In a preferred embodiment of the present disclosure, the spacer layer is of polyester, such as a 3D mesh polyester fabric.

In an embodiment of the present disclosure, the wound dressing comprises an absorbent layer configured for retaining wound exudate. The absorbent layer may comprise a number of layers comprising super absorbent particles. In specific embodiments, the absorbent layer comprises two, or three, or four, super absorbent layers. It is a preference that the absorbent layer is directly below the cover layer. It is a preference that the absorbent layer comprises granulated super absorbent particles. Preferably the absorbent layer comprises an aperture, i.e. an absorbent layer aperture. It is a further preference that at least a part of said absorbent layer aperture overlaps the cover layer aperture, thereby forming a through hole through the absorbent layer and the cover layer.

Super-absorber particles/fibers may be, for example, sodium polyacrylate or carbomethoxycellulose materials or the like or any material capable of absorbing many times its own weight in liquid. In some embodiments, the material can absorb more than five times its own weight of 0.9 percent W/W saline, etc. In some embodiments, the material can absorb more than 15 times its own weight of 0.9 percent W/W saline, etc. In some embodiments, the material is capable of absorbing more than 20 times its own weight of 0.9 percent W/W saline, etc. Preferably, the material is capable of absorbing more than 30 times its own weight of 0.9 percent W/W saline, etc.

In a preferred embodiment the dressing comprises a cover layer in a liquid impermeable and gas permeable material. For example, the material of the cover layer may be polyurethane. The cover layer may comprise an adhesive for contacting the layer below the cover layer, such as the absorbent layer. In a preferred embodiment of the present disclosure, the cover layer comprises an aperture, i.e. a cover layer aperture. In specific embodiments of the present disclosure, the cover layer aperture is aligned with an absorbent layer aperture, such as aligned around a vertical axis, such as an axis perpendicular to the plane of the dressing. Thereby, the cover layer aperture may be centered around a vertical axis perpendicular to the plane of the dressing. In general embodiments of the present disclosure, the cover layer aperture is aligned with the filter element, and the sealing layer aperture, such as wherein each of the filter element, the cover layer aperture and the sealing layer aperture are centered around an axis perpendicular to the plane of the dressing.

The dressing comprises a sealing layer adhered to the cover layer, such as the top surface of the cover layer facing away from the wound contacting layer, or a bottom surface of the cover layer facing the wound contacting layer. The sealing layer comprises a sealing layer aperture, the sealing layer aperture being in fluidic communication with the cover layer aperture. The sealing layer may further comprise adhesives, or the sealing layer may be provided in an adhering material, to adhere to other layers of the dressing.

The filter element is preferably impermeable to liquids, but permeable to gases. Preferably the filter element is provided to act as a liquid barrier and to ensure that no liquids are able to escape from the wound dressing. The filter element may also function as a bacterial barrier. The filter element may be provided with a pore size of 0.2 µm or less, such as 0.1 µm or less, such as 0.05 µm or less, or even 0.01 µm or less. In a preferred embodiment of the present disclosure, the filter element is provided in a paper material. The filter element can be attached or sealed to the cover layer and/or sealing layer. Preferably, the filter is arranged to cover the sealing layer aperture. The filter may be provided in a circular, or oval shape, for example a circular shape with a diameter of between 5 mm and 20 mm, such as between 7 mm and 18 mm, such as between 9 mm and 17 mm, such as between 10 mm and 16 mm, such as around 13 mm. In a further embodiment of the present disclosure the wound dressing is configured as disclosed elsewhere herein.

The air connector comprises a proximal end configured for connecting to a negative pressure source, and a distal end configured for connecting to the wound dressing. The proximal end may for example comprise a connector, for connecting to a tubing or a negative pressure source. The connector may extend from the proximal end of the wound treatment apparatus. The connector may be configured for easy connection to other components of a NPWT system, such as a negative pressure source, and said connector may comprise a Luer-lock or similar connection system, for easy connection.

In an embodiment of the present disclosure, the top layer of the air connector and the bottom layer of the air connector are adhered together forming a sealed environment. The top and bottom layers may for example be welded together, or adhesives may have been used in order to adhere the top and bottom layers of the air connector.

In a preferred embodiment of the present disclosure, the air connector comprises a connector spacer layer between the top and bottom layers. The connector spacer layer is preferably provided in a porous material. The connector spacer layer is preferably configured such that negative pressure may be transmitted through the connector spacer layer. The connector spacer layer is preferably provided in the sealed environment, formed by the connector top layer and the connector bottom layer, such as wherein the connector spacer layer is encapsulated by the connector top layer and the connector bottom layer.

In an embodiment of the present disclosure, the air connector comprises an opening at a distal end of the air connector. The opening is preferably provided in the bottom layer of the air connector. Preferably, the wound treatment apparatus is configured such that the air connector is attached to the dressing such that the opening of the air connector forms a sealed fluid connection to the cover layer aperture of the dressing. The air connector is thereby configured to connect to the dressing such that a sealed fluid connection is formed between the sealed environments of the air connector and the dressing. It is a preference that the wound treatment apparatus is configured such that a sealed flow channel is formed between the sealed environment of the dressing and the sealed environment of the air connector.

In an embodiment of the present disclosure, the surface surrounding the opening of the air connector is attached, such as adhered, to a surface surrounding the cover layer aperture. Typically, the bottom surface of the air connector comprises the opening and the air connector is attached to the dressing such that the bottom surface of the air connector connects to the top surface of the cover layer. However, in specific embodiments of the present disclosure, there may be layers between the cover layer of the dressing and the bottom layer of the air connector. In a typical embodiment of the present disclosure, the bottom layer of the air connector is attached, such as adhered, to a part of the top surface of a sealing layer, wherein the sealing layer is above the cover layer. The sealing layer and/or the cover layer may comprise adhesive layers for attaching to the air connector. In a preferred embodiment of the present disclosure, the wound treatment apparatus further comprises a negative pressure source, such as a pump unit. It is a preference that the wound treatment apparatus is arranged such that the negative pressure source provides negative pressure to the wound dressing, through the air connector.

### Detailed description of drawings

The drawings are exemplary and are intended to illustrate some of the features of the presently disclosed wound dressing and wound treatment apparatus, and are not to be construed as limiting to the presently disclosed invention.

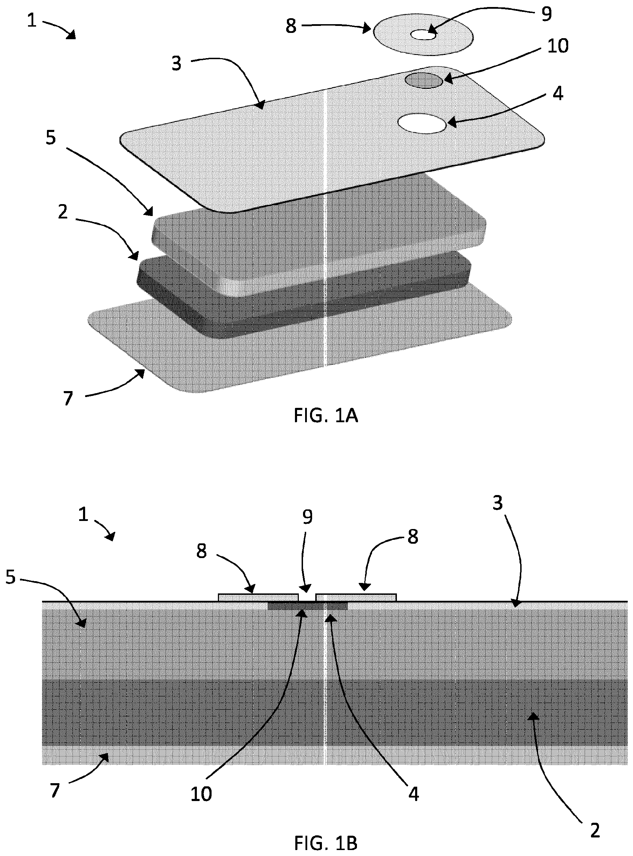

Fig. 1A shows a schematic illustration, in exploded view, of a wound dressing (1) for use in negative pressure wound therapy according to a specific embodiment of the present disclosure. The wound dressing comprises a spacer layer (2), a cover layer (3), an absorbent layer (5), a sealing layer (8) and a filter element (10). The cover layer (3) comprising a cover layer aperture (4). The sealing layer (8) is adhered to the top surface of the cover layer, the sealing layer comprising a sealing layer aperture (9), the sealing layer aperture (9) being in fluidic communication with the cover layer aperture(4). The filter element (10) is arranged to cover the sealing layer aperture. The wound dressing further comprises a wound contacting layer (7).

Fig. 1B shows a schematic illustration of a central part of a cross section of a wound dressing for use in negative pressure wound therapy according to Fig. 1A. The wound dressing comprises a spacer layer (2), a cover layer (3), an absorbent layer (5), a sealing layer (8) and a filter element (10). The cover layer (3) comprising a cover layer aperture (4). The sealing layer (8) is adhered to the top surface of the cover layer, the sealing layer comprising a sealing layer aperture (9), the sealing layer aperture (9) being in fluidic communication with the cover layer aperture(4). The filter element (10) is arranged to cover the sealing layer aperture. The wound dressing further comprises a wound contacting layer (7). It can be seen that the filter element is located in the cover layer aperture, covered by the sealing layer aperture. The filter element is above the absorbent layer, in the shown embodiment, the filter element is in contact with the absorbent layer, however it may be advantageous to have a gap between the absorbent layer and the filter element, as disclosed elsewhere herein. For example a gap of around 25 mm between the filter element and the absorbent layer. This may be accomplished by the use of distancing units between the filter element and the absorbent unit.

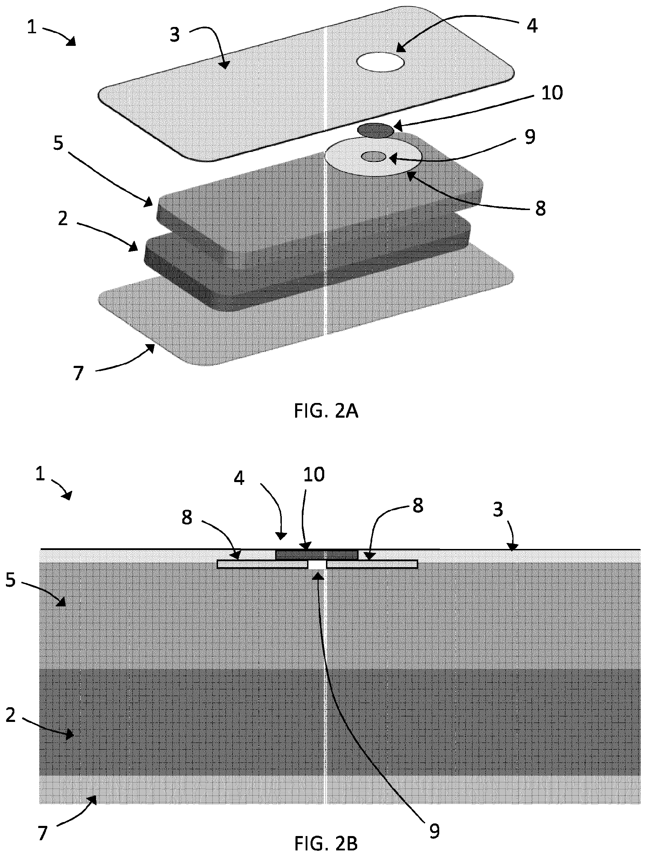

Fig. 2A shows a schematic illustration, in exploded view, of a wound dressing (1) for use in negative pressure wound therapy according to a specific embodiment of the present disclosure. The wound dressing comprises a spacer layer (2), a cover layer (3), an absorbent layer (5), a sealing layer (8), a filter element (10) and a wound contacting layer. The cover layer (3) comprising a cover layer aperture (4). The sealing layer (8) is adhered to the bottom surface of the cover layer, the sealing layer comprising a sealing layer aperture (9), the sealing layer aperture (9) being in fluidic communication with the cover layer aperture(4). The filter element (10) is arranged to cover the sealing layer aperture.

Fig. 2B shows a schematic illustration of a central part of a cross section of a wound dressing for use in negative pressure wound therapy according to Fig. 2A. The wound dressing comprises a spacer layer (2), a cover layer (3), an absorbent layer (5), a sealing layer (8) and a filter element (10). The cover layer (3) comprising a cover layer aperture (4). The sealing layer (8) is adhered to the bottom surface of the cover layer, the sealing layer comprising a sealing layer aperture (9), the sealing layer aperture (9) being in fluidic communication with the cover layer aperture(4). The filter element (10) is arranged to cover the sealing layer aperture. The wound dressing further comprises a wound contacting layer (7). It can be seen that the filter element is located in the cover layer aperture, covered by the sealing layer aperture.

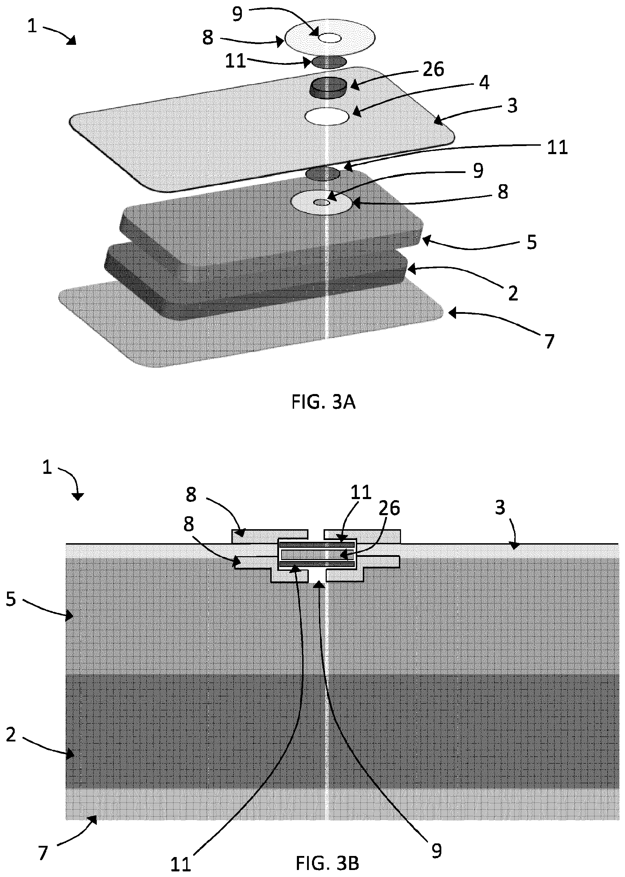

Fig. 3A shows a schematic illustration, in exploded view, of a wound dressing (1) for use in negative pressure wound therapy according to a specific embodiment of the present disclosure. The wound dressing comprises a wound contacting layer (7), a spacer layer (2), a cover layer (3) comprising a cover layer aperture (4), and an absorbent layer (5) wherein the absorbent layer is positioned above the wound contacting layer (7). Typically the cover layer is attached to the wound contacting layers, thereby a sealed environment is formed encapsulating the absorbent layer and the spacer layer. The exemplified wound dressing shown, comprises multiple sealing layers (8), in the shown example two sealing layers. One sealing layer attaches to the top surface of the cover layer while the other attaches to the bottom of the cover layer. The sealing layers encapsulate a filter element (10) comprising multiple layers. The shown filter element comprises a hydrophilic layer (26), for example wherein the material of said filter comprises or consists of super absorbent polymers. The hydrophilic layer is encapsulated by two hydrophobic layers (11).

Fig. 3B shows a schematic illustration of a central part of a cross section of a wound dressing for use in negative pressure wound therapy according to Fig. 3A. The wound dressing comprises a wound contacting layer (7), a spacer layer (2), a cover layer (3) comprising a cover layer aperture (4), and an absorbent layer (5) wherein the absorbent layer is positioned above the wound contacting layer (7). Typically the cover layer is attached to the wound contacting layers, thereby a sealed environment is formed encapsulating the absorbent layer and the spacer layer. The exemplified wound dressing shown, comprises multiple sealing layers (8), in the shown example two sealing layers. One sealing layer attaches to the top surface of the cover layer while the other attaches to the bottom of the cover layer. The sealing layers encapsulate a filter element (10) comprising multiple layers. The shown filter element comprises a hydrophilic layer (26), for example wherein the material of said filter comprises or consists of super absorbent polymers. The hydrophilic layer is encapsulated by two hydrophobic layers (11).

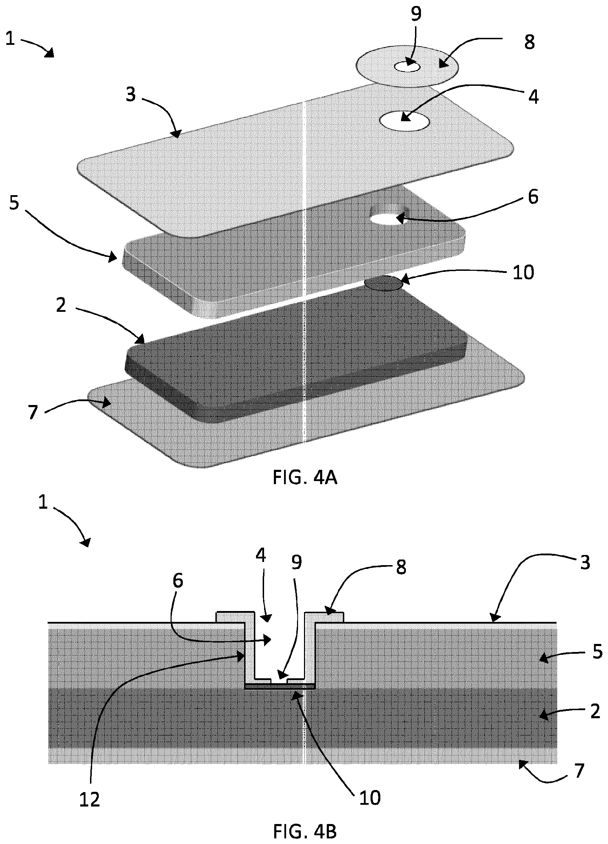

Fig. 4A shows a schematic illustration, in exploded view, of a wound dressing (1) for use in negative pressure wound therapy according to a specific embodiment of the present disclosure. The exemplified wound dressing (1) comprises a cover layer (3) attached to the wound contacting layer (7), such as welded at a peripheral part, typically along a perimeter of the wound dressing, thereby forming a sealed environment that encapsulates the spacer layer (2), and the absorbent layer (5). The wound dressing comprises a sealing layer (8) that is attached to a top surface of the cover layer (3), around the cover layer aperture (4). The sealing layer may however additionally or alternatively be attached to the bottom layer of the cover layer. The sealing layer may be provided as a planar unit, however following manufacturing of the wound dressing, the sealing layer is to extend from the cover layer aperture down, towards the wound contacting layer (7) through an absorbent layer aperture (6) formed in the absorbent layer (5). The sealing layer forms a conduit through the absorbent layer aperture. At a bottom part of the sealing layer, towards the wound contacting layer, there is a sealing layer aperture (9) that is covered by a filter element (10). In the specific embodiment shown, the filter element is attached to a bottom surface of the sealing layer, but the filter element may be attached to a top surface of the sealing layer, such as within the conduit formed by the sealing layer. Typically, the sealing layer aperture, and/or the cover layer aperture are oval or circular, however in specific embodiments of the present disclosure, said apertures may be provided in any shape such as irregular or regular, for example a polygonal shape. The sealing layer may be sealed around the cover layer aperture, for example in a ring pattern, and have a sealing layer aperture within the area sealed to the cover layer. The sealing layer is preferably arranged to be impermeable to air and water vapour. The material of the sealing layer is preferably hydrocolloid or any other material that can be arranged to be impermeable to air and water vapour, such as silicone, polyurethan, thermoplastic polyurethane or any other plastic material that may be arranged to be impermeable to air and water vapour.

Fig. 4B shows a schematic illustration of a central part of a cross section of a wound dressing for use in negative pressure wound therapy according to Fig. 4A. The figure shows the central part of the wound dressing (1), typically the cover layer (3) and the wound contacting layer (7) are attached, such as welded, at a peripheral part, typically along a perimeter of the wound dressing, thereby forming a sealed environment that encapsulates the spacer layer (2), and the absorbent layer (5). The wound dressing comprises a sealing layer (8) that is attached to a top surface of the cover layer (3), around the cover layer aperture (4). The sealing layer extends from the cover layer aperture down, towards the wound contacting layer (7) through an absorbent layer aperture (6) formed in the absorbent layer (5). The sealing layer forms a conduit (12) through the absorbent layer aperture. At a bottom part of the sealing layer, towards the wound contacting layer, there is a sealing layer aperture (9) that is covered by a filter element (10). In the specific embodiment shown, the filter element is attached to a bottom surface of the sealing layer, but the filter element may be attached to a top surface of the sealing layer, such as within the conduit formed by the sealing layer. Typically, the sealing layer aperture, and/or the cover layer aperture are oval or circular, however in specific embodiments of the present disclosure, said apertures may be provided in any shape such as irregular or regular, for example a polygonal shape. The sealing layer may be sealed around the cover layer aperture, for example in a ring pattern, and have a sealing layer aperture within the area sealed to the cover layer. The sealing layer is preferably arranged to be impermeable to air and water vapour. The material of the sealing layer is preferably hydrocolloid or any other material that can be arranged to be impermeable to air and water vapour, such as silicone, polyurethan, thermoplastic polyurethane or any other plastic material that may be arranged to be impermeable to air and water vapour.

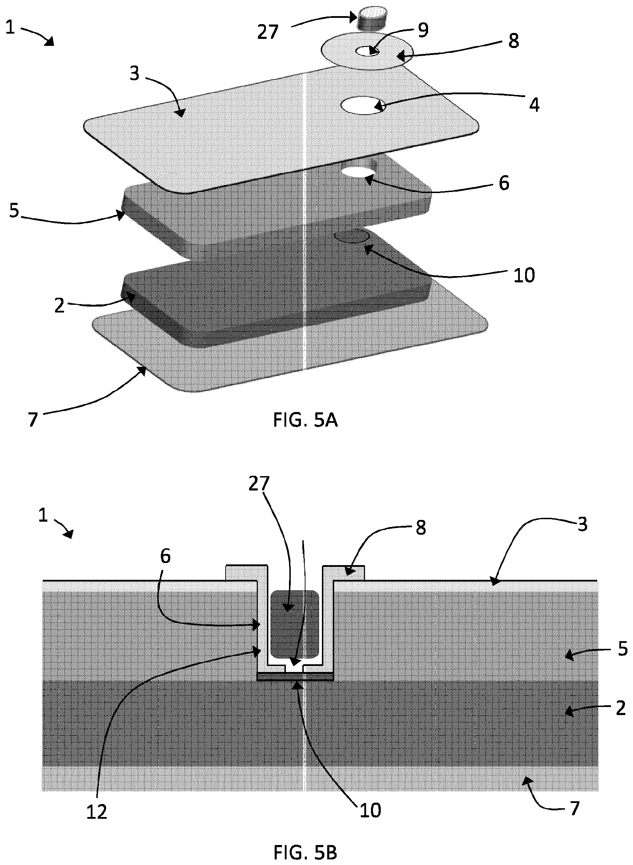

Fig. 5A shows a schematic illustration, in exploded view, of a wound dressing (1) for use in negative pressure wound therapy according to a specific embodiment of the present disclosure. The exemplified wound dressing (1) comprises a cover layer (3) attached to the wound contacting layer (7), such as welded at a peripheral part, typically along a perimeter of the wound dressing, thereby forming a sealed environment that encapsulates the spacer layer (2), and the absorbent layer (5). The wound dressing comprises a sealing layer (8) that is attached to a top surface of the cover layer (3), around the cover layer aperture (4). The sealing layer may however additionally or alternatively be attached to the bottom layer of the cover layer. The sealing layer may be provided as a planar unit, however following manufacturing of the wound dressing, the sealing layer is to extend from the cover layer aperture down, towards the wound contacting layer (7) through an absorbent layer aperture (6) formed in the absorbent layer (5). The sealing layer forms a conduit through the absorbent layer aperture. At a bottom part of the sealing layer, towards the wound contacting layer, there is a sealing layer aperture (9) that is covered by a filter element (10). In the specific embodiment shown, the filter element is attached to a bottom surface of the sealing layer, but the filter element may be attached to a top surface of the sealing layer, such as within the conduit formed by the sealing layer. Typically, the sealing layer aperture, and/or the cover layer aperture are oval or circular, however in specific embodiments of the present disclosure, said apertures may be provided in any shape such as irregular or regular, for example a polygonal shape. The sealing layer may be sealed around the cover layer aperture, for example in a ring pattern, and have a sealing layer aperture within the area sealed to the cover layer. The sealing layer is preferably arranged to be impermeable to air and water vapour. The material of the sealing layer is preferably hydrocolloid or any other material that can be arranged to be impermeable to air and water vapour, such as silicone, polyurethan, thermoplastic polyurethane or any other plastic material that may be arranged to be impermeable to air and water vapour. The wound dressing comprises a filler unit (27) located in the conduit formed by the sealing layer. The filler unit may comprise or consist of a hydrophilic and/or a hydrophobic material. For example the filler unit may consist of a hydrophilic material, such as polyvinyl alcohol and/or super absorbent polymers. This enables a wound dressing that restricts the supply of negative pressure to the wound dressing, such as from a negative pressure source. The restriction of supply of negative pressure may be detected by the use of an alarm system, such as by a unit comprising the negative pressure source. The alarm system may monitor, continuously, periodically, randomly, or at specific time points, the pressure in the system, such as at an air connector between the wound dressing and the negative pressure source. If a blockage is detected, an alarm may be provided to a user that the wound dressing and/or the air connector may need replacement. In other embodiments of the present disclosure, the filler unit may comprise or consist of a hydrophobic material. The use of a hydrophobic material may prolong the time or operation as it may prevent unwanted fluid, such as wound exudate liquid, from entering the air connector and/or the negative pressure source. A hydrophobic filler unit acts to retain wound exudate in the wound dressing, thereby increasing the time until the wound dressing requires replacement.

Fig. 5B shows a schematic illustration of a central part of a cross section of a wound dressing for use in negative pressure wound therapy according to Fig. 5A. The figure shows the central part of the wound dressing (1), typically the cover layer (3) and the wound contacting layer (7) are attached, such as welded, at a peripheral part, typically along a perimeter of the wound dressing, thereby forming a sealed environment that encapsulates the spacer layer (2), and the absorbent layer (5). The wound dressing comprises a sealing layer (8) that is attached to a top surface of the cover layer (3), around the cover layer aperture (4). The sealing layer extends from the cover layer aperture down, towards the wound contacting layer (7) through an absorbent layer aperture (6) formed in the absorbent layer (5). The sealing layer forms a conduit (12) through the absorbent layer aperture. At a bottom part of the sealing layer, towards the wound contacting layer, there is a sealing layer aperture (9) that is covered by a filter element (10). In the specific embodiment shown, the filter element is attached to a bottom surface of the sealing layer, but the filter element may be attached to a top surface of the sealing layer, such as within the conduit formed by the sealing layer. Typically, the sealing layer aperture, and/or the cover layer aperture are oval or circular, however in specific embodiments of the present disclosure, said apertures may be provided in any shape such as irregular or regular, for example a polygonal shape. The sealing layer may be sealed around the cover layer aperture, for example in a ring pattern, and have a sealing layer aperture within the area sealed to the cover layer. The sealing layer is preferably arranged to be impermeable to air and water vapour, however the sealing layer may be arranged to be permeable to gasses and impermeable to liquids, as it may facilitate removal of wound exudate. The material of the sealing layer is preferably hydrocolloid or any other material that can be arranged to be impermeable to air and water vapour, such as silicone, polyurethan, thermoplastic polyurethane or any other plastic material that may be arranged to be impermeable to air and water vapour. The wound dressing comprises a filler unit (27) located in the conduit formed by the sealing layer.

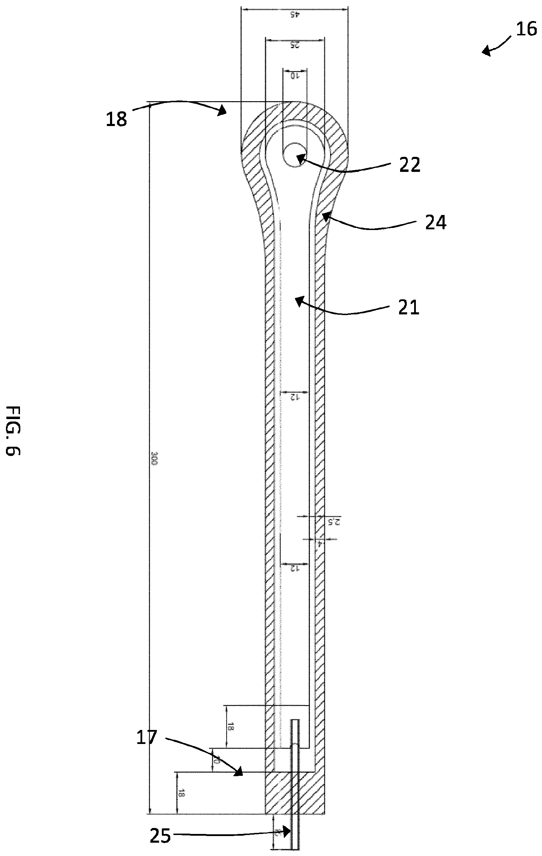

Fig. 6 shows an air connector (16) for use in negative pressure wound therapy according to a specific embodiment of the present disclosure. The air connector comprises a proximal end (17) configured for connecting to a negative pressure source, and a distal end (18) configured for connecting to the wound dressing. The proximal end may for example comprise a connector (25), for connecting to a tubing or a negative pressure source. The connector may extend from the proximal end of the wound treatment apparatus. The connector may be configured for easy connection to other components of a NPWT system, such as a negative pressure source, and said connector may comprise a Luer-lock or similar connection system, for easy connection. The top layer of the air connector and the bottom layer of the air connector are attached together at a peripheral part (24) of the air connector, typically along a perimeter of the air connector, forming a sealed environment encapsulating a connector spacer layer (21). The air connector comprises an opening (22) at a distal end of the air connector. The opening is preferably provided in the bottom layer of the air connector. Preferably, the wound treatment apparatus is configured such that the air connector is attached to the dressing such that the opening of the air connector forms a sealed fluid connection to the cover layer aperture of the dressing. The air connector is thereby configured to connect to the dressing such that a sealed fluid connection is formed between the sealed environments of the air connector and the dressing.

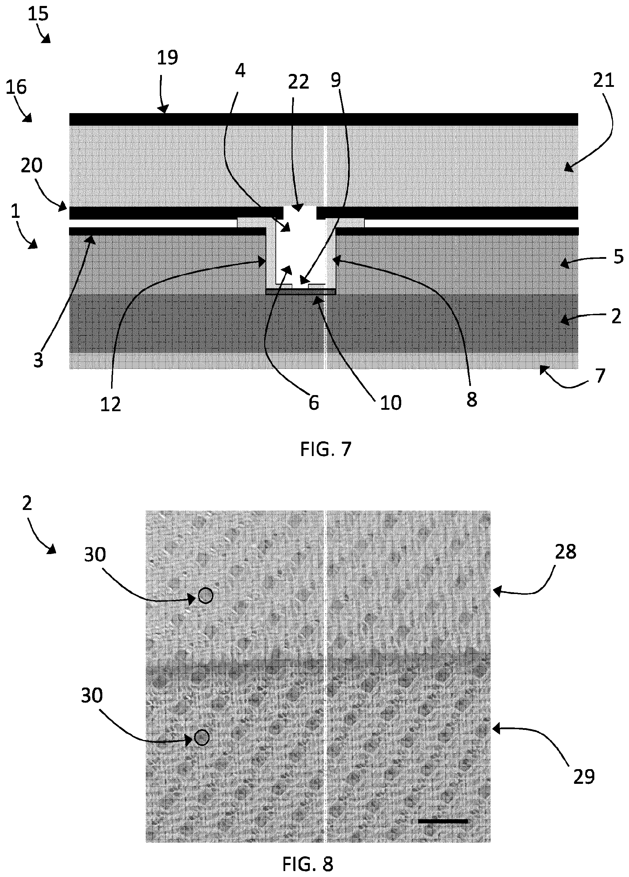

Fig. 7 shows a cross section of a central part of a wound treatment apparatus (15) for use in negative pressure wound therapy according to a specific embodiment of the present disclosure. The wound treatment apparatus (15) comprises an air connector (16) and a wound dressing (1). The air connector is, at a distal end, configured for connecting to the wound dressing. The top layer (19) of the air connector and the bottom layer (20) of the air connector are attached together at a peripheral part of the air connector, typically along a perimeter of the air connector, forming a sealed environment encapsulating a connector spacer layer (21). The air connector comprises an opening (22) at a distal end of the air connector. The opening is preferably provided in the bottom layer of the air connector. Preferably, the wound treatment apparatus is configured such that the air connector is attached to the dressing such that the opening of the air connector forms a sealed fluid connection to the cover layer aperture of the dressing. The air connector is thereby configured to connect to the dressing such that a sealed fluid connection is formed between the sealed environments of the air connector and the dressing. The figure shows an exemplified wound dressing, the wound treatment apparatus may comprise other types of wound dressings as disclosed elsewhere herein. The exemplified wound dressing comprises a wound contacting layer (7), a spacer layer (2), a cover layer (3) comprising a cover layer aperture (4), an absorbent layer (5) positioned above the wound contacting layer (7), the absorbent layer comprising an absorbent layer aperture (6), a sealing layer (8) adhered to the cover layer (3). The sealing layer comprises a sealing layer aperture (9), the sealing layer aperture being in fluidic communication with the cover layer aperture (4), and a filter element (10) arranged to cover the sealing layer aperture.

Fig. 8 shows a top layer (28) and a bottom layer (29) of the spacer layer (2) according to a specific embodiment of the present disclosure. The scale bar in the bottom right is approximately 5 mm. The spacer layer is provided in a knitted material forming a plurality of larger holes (30) of substantially the same size on both layers. The holes in the shown example are approximately 1400 µm. The spacer layer is provided with a top and bottom layer connected by vertical filaments, and wherein the top and bottom layer comprises holes of the same size. Similar sized holes of the top layer and the bottom layer of the spacer layer may have multiple advantages. For example, it is given that a larger size of the holes facing the wound contacting layer offer a greater chance to allow semi-solid particles released from sluff, biofilm and other harmful material to be separated from the wound surface and pass through the spacer layer into an upper layer of the wound dressing, such as the absorbent layer. This is in contrast to a spacer layer in which its bottom layer has smaller holes compared to the holes at its top layer. Additional advantages of a spacer layer with similar sized holes is that particulate matter, such as blood agglutinates, does not become retained within the spacer layer. In a preferred embodiment of the present disclosure, the spacer layer is of polyester, such as a 3D mesh polyester fabric.

## Claims

1. A wound dressing (1) for use in negative pressure wound therapy of a wound site, the wound dressing (1) comprising:
• a wound contacting layer (7) comprising multiple perforations;
• a spacer layer (2), above the wound contacting layer (7), configured for transmission of wound exudate away from the wound site;
• an absorbent layer (5), above the spacer layer (2);
• a cover layer (3) comprising a cover layer aperture (4), and wherein the cover layer (3) is sealed to the wound contacting layer (7) along a peripheral part of the wound dressing (1), thereby forming a sealed environment comprising the spacer layer (2) and the absorbent layer (5);
• at least one sealing layer (8), sealed to the cover layer (3) around the cover layer aperture (4), the at least one sealing layer (8) comprising a sealing layer aperture (9), the sealing layer aperture (9) being in fluidic communication with the cover layer aperture (4); and
• a filter element (10), located in, or below, the cover layer aperture (4) and above the spacer layer (2), and wherein the filter element (10) is retained by the sealing layer (8) and arranged to cover the sealing layer aperture (9);
wherein the absorbent layer (5) comprises an absorbent layer aperture (6) in fluidic connection with the cover layer aperture (4); and
wherein the at least one sealing layer (8) forms a conduit (12) that extends from the part of the cover layer (3) that is sealed to the sealing layer (8), towards the bottom of the absorbent layer (5) through at least a part of the absorbent layer aperture (6).

2. The wound dressing (1) according to claim 1,
wherein the filter element (10) is located below the cover layer aperture (4), within the sealed environment, and is arranged to cover the cover layer aperture (4); and/or
wherein the filter element (10) is located between the sealing layer (8) and the spacer layer (2), such as between the sealing layer aperture (9) and the spacer layer (2); and/or wherein the filter element (10) is located in or below the absorbent layer aperture (6).

3. The wound dressing (1) according to any one of the preceding claims, wherein the filter element (10) comprises a first side and a second side, and wherein the first side has a smoother surface than the second side; and wherein
the filter element (10) is arranged such that the first side faces away from the wound contacting layer (7) and the second side faces the wound contacting layer (7).

4. The wound dressing (1) according to claim 3, wherein the sealing layer (8) is arranged to retain the filter element (10) by contacting the first side of the filter element (10).

5. The wound dressing (1) according to any one of the preceding claims, wherein the filter element (10) is attached, to a top surface and/or a bottom surface of the at least one sealing layer (8), such as wherein
wherein the filter element (10) is laminated between a first and a second sealing layer (8) comprising at least partially overlapping sealing layer apertures (9).

6. The wound dressing (1) according to any one of the preceding claims, wherein the filter element (10) comprises multiple layers of different hydrophobic properties, such as
wherein the filter element (10) comprises or consists of a hydrophilic layer between two hydrophobic layers.

7. The wound dressing (1) according to any one of the preceding claims, wherein the absorbent layer aperture (6) is covered by the cover layer aperture (4), and wherein the absorbent layer aperture (6) extends from a top surface of the absorbent layer (5) towards the wound contacting layer (7), through at least a part of the absorbent layer (5), such as
wherein the absorbent layer aperture (6) extends through the thickness of the absorbent layer (5).

8. The wound dressing (1) according to any one of the preceding claims, wherein the conduit (12) extends to the bottom of the absorbent layer (5).

9. The wound dressing (1) according to any one of the preceding claims, wherein the sealing layer aperture (9) forms an opening at the bottom of the conduit (12).

10. The wound dressing (1) according to any one of the preceding claims, wherein the sealing layer (8) is impermeable to liquids, preferably at least water.

11. The wound dressing according to claim 10, wherein the sealing layer is impermeable to air and/or water vapour.

12. The wound dressing (1) according to any one of the preceding claims, comprising a top sealing layer (8) and a bottom sealing layer (8), and wherein the sealing layers (8) encapsulate the filter element (10) such that the sealing layer apertures (9) are located on each side of the filter element (10).

13. The wound dressing (1) according to claim 12, wherein the top sealing layer (8) adheres to an outer part of the cover layer (3) and a bottom sealing layer (8) adheres to an inner part of the cover layer (3).

14. The wound dressing (1) according to any one of the preceding claims, wherein the sealing layer (8) comprises or consists of a hydrophobic material, such as hydrocolloid.

15. A wound treatment apparatus (15) for use in a canister-free negative pressure wound therapy, the wound treatment apparatus (15) comprising:
• a wound dressing (1) according to any one of the preceding claims;
• an air connector (16) adapted to provide negative pressure, from a negative pressure source, to the wound dressing (1), through the cover layer aperture (4), the air connector (16) comprising:
- a proximal end (17) configured for connecting to a negative pressure source, and a distal end (18) configured for connecting to the wound dressing (1);
- a top layer (19) and a bottom layer (20) that are adhered together forming a sealed environment;
- a connector spacer layer (21) between the top and bottom layers (19, 20);
- an opening (22) at the distal end (18) that is positioned above the cover layer aperture (4), such that a sealed flow channel between the sealed environment of the dressing and the sealed environment of the air connector is formed.

## Patentansprüche

1. Wundverband (1) zur Verwendung in der Unterdruck-Wundtherapie einer Wundstelle, wobei der Wundverband (1) umfasst: · eine wundkontaktierende Schicht (7) mit mehreren Perforationen; · eine Abstandsschicht (2), oberhalb der wundkontaktierenden Schicht (7), ausgelegt zur Übertragung von Wundexsudat von der Wundstelle weg; · eine Absorptionsschicht (5), oberhalb der Abstandsschicht (2); · eine Deckschicht (3) mit einer Deckschichtöffnung (4), wobei die Deckschicht (3) entlang eines peripheren Teils des Wundverbands (1) mit der wundkontaktierenden Schicht (7) versiegelt ist, wodurch eine abgedichtete Umgebung gebildet wird, die die Abstandsschicht (2) und die Absorptionsschicht (5) umfasst; . mindestens eine Dichtungsschicht (8), die um die Deckschichtöffnung (4) herum mit der Deckschicht (3) versiegelt ist, wobei die mindestens eine Dichtungsschicht (8) eine Dichtungsschichtöffnung (9) umfasst, wobei die Dichtungsschichtöffnung (9) in fluidischer Verbindung mit der Deckschichtöffnung (4) steht; und · ein Filterelement (10), das in oder unterhalb der Deckschichtöffnung (4) und oberhalb der Abstandsschicht (2) angeordnet ist, wobei das Filterelement (10) von der Dichtungsschicht (8) gehalten wird und so angeordnet ist, dass es die Dichtungsschichtöffnung (9) abdeckt; wobei die Absorptionsschicht (5) eine Absorptionsschichtöffnung (6) in fluidischer Verbindung mit der Deckschichtöffnung (4) umfasst; und wobei die mindestens eine Dichtungsschicht (8) einen Kanal (12) bildet, der sich von dem Teil der Deckschicht (3), der mit der Dichtungsschicht (8) versiegelt ist, in Richtung des Bodens der Absorptionsschicht (5) durch mindestens einen Teil der Absorptionsschichtöffnung (6) erstreckt.

2. Wundverband (1) nach Anspruch 1, wobei das Filterelement (10) unterhalb der Deckschichtöffnung (4), innerhalb der abgedichteten Umgebung, angeordnet ist und so angeordnet ist, dass es die Deckschichtöffnung (4) abdeckt; und/oder wobei das Filterelement (10) zwischen der Dichtungsschicht (8) und der Abstandsschicht (2) angeordnet ist, wie beispielsweise zwischen der Dichtungsschichtöffnung (9) und der Abstandsschicht (2); und/oder wobei das Filterelement (10) in oder unterhalb der Absorptionsschichtöffnung (6) angeordnet ist.

3. Wundverband (1) nach einem der vorhergehenden Ansprüche, wobei das Filterelement (10) eine erste Seite und eine zweite Seite umfasst, und wobei die erste Seite eine glattere Oberfläche als die zweite Seite aufweist; und wobei das Filterelement (10) so angeordnet ist, dass die erste Seite von der wundkontaktierenden Schicht (7) weg weist und die zweite Seite zur wundkontaktierenden Schicht (7) hin weist.

4. Wundverband (1) nach Anspruch 3, wobei die Dichtungsschicht (8) so angeordnet ist, dass sie das Filterelement (10) durch Kontaktieren der ersten Seite des Filterelements (10) hält.

5. Wundverband (1) nach einem der vorhergehenden Ansprüche, wobei das Filterelement (10) an einer oberen Oberfläche und/oder einer unteren Oberfläche der mindestens einen Dichtungsschicht (8) befestigt ist, wie beispielsweise wobei das Filterelement (10) zwischen einer ersten und einer zweiten Dichtungsschicht (8) laminiert ist, die zumindest teilweise überlappende Dichtungsschichtöffnungen (9) umfassen.

6. Wundverband (1) nach einem der vorhergehenden Ansprüche, wobei das Filterelement (10) mehrere Schichten mit unterschiedlichen hydrophoben Eigenschaften umfasst, wie beispielsweise wobei das Filterelement (10) eine hydrophile Schicht zwischen zwei hydrophoben Schichten umfasst oder daraus besteht.

7. Wundverband (1) nach einem der vorhergehenden Ansprüche, wobei die Absorptionsschichtöffnung (6) von der Deckschichtöffnung (4) abgedeckt ist, und wobei sich die Absorptionsschichtöffnung (6) von einer oberen Oberfläche der Absorptionsschicht (5) in Richtung der wundkontaktierenden Schicht (7) durch mindestens einen Teil der Absorptionsschicht (5) erstreckt, wie beispielsweise wobei sich die Absorptionsschichtöffnung (6) durch die Dicke der Absorptionsschicht (5) erstreckt.

8. Wundverband (1) nach einem der vorhergehenden Ansprüche, wobei sich der Kanal (12) bis zum Boden der Absorptionsschicht (5) erstreckt.

9. Wundverband (1) nach einem der vorhergehenden Ansprüche, wobei die Dichtungsschichtöffnung (9) eine Öffnung am Boden des Kanals (12) bildet.

10. Wundverband (1) nach einem der vorhergehenden Ansprüche, wobei die Dichtungsschicht (8) für Flüssigkeiten undurchlässig ist, vorzugsweise zumindest für Wasser.

11. Wundverband nach Anspruch 10, wobei die Dichtungsschicht für Luft und/oder Wasserdampf undurchlässig ist.

12. Wundverband (1) nach einem der vorhergehenden Ansprüche, umfassend eine obere Dichtungsschicht (8) und eine untere Dichtungsschicht (8), wobei die Dichtungsschichten (8) das Filterelement (10) derart einkapseln, dass die Dichtungsschichtöffnungen (9) auf jeder Seite des Filterelements (10) angeordnet sind.

13. Wundverband (1) nach Anspruch 12, wobei die obere Dichtungsschicht (8) an einem äußeren Teil der Deckschicht (3) haftet und eine untere Dichtungsschicht (8) an einem inneren Teil der Deckschicht (3) haftet.

14. Wundverband (1) nach einem der vorhergehenden Ansprüche, wobei die Dichtungsschicht (8) ein hydrophobes Material umfasst oder daraus besteht, wie beispielsweise Hydrocolloid.

15. Wundbehandlungsapparat (15) zur Verwendung in einer kanisterlosen Unterdruck-Wundtherapie, wobei der Wundbehandlungsapparat (15) umfasst: · einen Wundverband (1) nach einem der vorhergehenden Ansprüche; · einen Luftanschluss (16), der angepasst ist, um Unterdruck von einer Unterdruckquelle zu dem Wundverband (1) durch die Deckschichtöffnung (4) bereitzustellen, wobei der Luftanschluss (16) umfasst: - ein proximales Ende (17), das zur Verbindung mit einer Unterdruckquelle ausgelegt ist, und ein distales Ende (18), das zur Verbindung mit dem Wundverband (1) ausgelegt ist; - eine obere Schicht (19) und eine untere Schicht (20), die miteinander verbunden sind und eine abgedichtete Umgebung bilden; - eine Anschluss-Abstandsschicht (21) zwischen der oberen und der unteren Schicht (19, 20); - eine Öffnung (22) am distalen Ende (18), die oberhalb der Deckschichtöffnung (4) positioniert ist, sodass ein abgedichteter Strömungskanal zwischen der abgedichteten Umgebung des Wundverbands und der abgedichteten Umgebung des Luftanschlusses gebildet wird.

## Revendications

1. Pansement (1) destiné à être utilisé dans une thérapie par pression négative d'un site de plaie, le pansement (1) comprenant : · une couche en contact avec la plaie (7) comprenant plusieurs perforations ; · une couche d'espacement (2), au-dessus de la couche en contact avec la plaie (7), configurée pour la transmission de l'exsudat de plaie loin du site de la plaie ; · une couche absorbante (5), au-dessus de la couche d'espacement (2) ; · une couche de couverture (3) comprenant une ouverture de couche de couverture (4), et dans laquelle la couche de couverture (3) est scellée à la couche en contact avec la plaie (7) le long d'une partie périphérique du pansement (1), formant ainsi un environnement scellé comprenant la couche d'espacement (2) et la couche absorbante (5) ; . au moins une couche d'étanchéité (8), scellée à la couche de couverture (3) autour de l'ouverture de couche de couverture (4), la au moins une couche d'étanchéité (8) comprenant une ouverture de couche d'étanchéité (9), l'ouverture de couche d'étanchéité (9) étant en communication fluidique avec l'ouverture de couche de couverture (4) ; et · un élément filtrant (10), situé dans, ou au-dessous de, l'ouverture de couche de couverture (4) et au-dessus de la couche d'espacement (2), et dans lequel l'élément filtrant (10) est retenu par la couche d'étanchéité (8) et agencé pour couvrir l'ouverture de couche d'étanchéité (9) ; dans lequel la couche absorbante (5) comprend une ouverture de couche absorbante (6) en connexion fluidique avec l'ouverture de couche de couverture (4) ; et dans lequel la au moins une couche d'étanchéité (8) forme un conduit (12) qui s'étend depuis la partie de la couche de couverture (3) qui est scellée à la couche d'étanchéité (8), vers le fond de la couche absorbante (5) à travers au moins une partie de l'ouverture de couche absorbante (6).

2. Pansement (1) selon la revendication 1, dans lequel l'élément filtrant (10) est situé au-dessous de l'ouverture de couche de couverture (4), à l'intérieur de l'environnement scellé, et est agencé pour couvrir l'ouverture de couche de couverture (4) ; et/ou dans lequel l'élément filtrant (10) est situé entre la couche d'étanchéité (8) et la couche d'espacement (2), par exemple entre l'ouverture de couche d'étanchéité (9) et la couche d'espacement (2) ; et/ou dans lequel l'élément filtrant (10) est situé dans ou au-dessous de l'ouverture de couche absorbante (6).

3. Pansement (1) selon l'une quelconque des revendications précédentes, dans lequel l'élément filtrant (10) comprend un premier côté et un second côté, et dans lequel le premier côté présente une surface plus lisse que le second côté ; et dans lequel l'élément filtrant (10) est agencé de telle sorte que le premier côté fasse face à l'opposé de la couche en contact avec la plaie (7) et que le second côté fasse face à la couche en contact avec la plaie (7).

4. Pansement (1) selon la revendication 3, dans lequel la couche d'étanchéité (8) est agencée pour retenir l'élément filtrant (10) en entrant en contact avec le premier côté de l'élément filtrant (10).

5. Pansement (1) selon l'une quelconque des revendications précédentes, dans lequel l'élément filtrant (10) est fixé, à une surface supérieure et/ou à une surface inférieure de la au moins une couche d'étanchéité (8), par exemple dans lequel l'élément filtrant (10) est laminé entre une première et une seconde couche d'étanchéité (8) comprenant des ouvertures de couche d'étanchéité (9) se chevauchant au moins partiellement.

6. Pansement (1) selon l'une quelconque des revendications précédentes, dans lequel l'élément filtrant (10) comprend plusieurs couches de propriétés hydrophobes différentes, par exemple dans lequel l'élément filtrant (10) comprend ou consiste en une couche hydrophile entre deux couches hydrophobes.

7. Pansement (1) selon l'une quelconque des revendications précédentes, dans lequel l'ouverture de couche absorbante (6) est couverte par l'ouverture de couche de couverture (4), et dans lequel l'ouverture de couche absorbante (6) s'étend depuis une surface supérieure de la couche absorbante (5) vers la couche en contact avec la plaie (7), à travers au moins une partie de la couche absorbante (5), par exemple dans lequel l'ouverture de couche absorbante (6) s'étend à travers l'épaisseur de la couche absorbante (5).

8. Pansement (1) selon l'une quelconque des revendications précédentes, dans lequel le conduit (12) s'étend jusqu'au fond de la couche absorbante (5).

9. Pansement (1) selon l'une quelconque des revendications précédentes, dans lequel l'ouverture de couche d'étanchéité (9) forme une ouverture au fond du conduit (12).

10. Pansement (1) selon l'une quelconque des revendications précédentes, dans lequel la couche d'étanchéité (8) est imperméable aux liquides, de préférence au moins à l'eau.

11. Pansement selon la revendication 10, dans lequel la couche d'étanchéité est imperméable à l'air et/ou à la vapeur d'eau.

12. Pansement (1) selon l'une quelconque des revendications précédentes, comprenant une couche d'étanchéité supérieure (8) et une couche d'étanchéité inférieure (8), et dans lequel les couches d'étanchéité (8) encapsulent l'élément filtrant (10) de telle sorte que les ouvertures de couche d'étanchéité (9) soient situées de chaque côté de l'élément filtrant (10).

13. Pansement (1) selon la revendication 12, dans lequel la couche d'étanchéité supérieure (8) adhère à une partie extérieure de la couche de couverture (3) et une couche d'étanchéité inférieure (8) adhère à une partie intérieure de la couche de couverture (3).

14. Pansement (1) selon l'une quelconque des revendications précédentes, dans lequel la couche d'étanchéité (8) comprend ou consiste en un matériau hydrophobe, tel qu'un hydrocolloïde.

15. Appareil de traitement de plaie (15) destiné à être utilisé dans une thérapie par pression négative sans cartouche, l'appareil de traitement de plaie (15) comprenant :
• un pansement (1) selon l'une quelconque des revendications précédentes ;
• un connecteur d'air (16) adapté pour fournir une pression négative, depuis une source de pression négative, au pansement (1), à travers l'ouverture de couche de couverture (4), le connecteur d'air (16) comprenant :
∘ une extrémité proximale (17) configurée pour être connectée à une source de pression négative, et une extrémité distale (18) configurée pour être connectée au pansement (1) ;
∘ une couche supérieure (19) et une couche inférieure (20) qui sont assemblées l'une à l'autre en formant un environnement scellé ;
∘ une couche d'espacement de connecteur (21) entre les couches supérieure et inférieure (19, 20) ;
∘ une ouverture (22) à l'extrémité distale (18) qui est positionnée au-dessus de l'ouverture de couche de couverture (4), de telle sorte qu'un canal d'écoulement scellé entre l'environnement scellé du pansement et l'environnement scellé du connecteur d'air soit formé.
